# EUROPÄISCHE PATENTSCHRIFT

(11) **EP 2 180 846 B1**
(45) Veröffentlichungstag und Bekanntmachung des Hinweises auf die Patenterteilung: **12.10.2011**
(21) Anmeldenummer: 08801123.4
(22) Anmeldetag: 04.08.2008
(51) Int. Cl.: A61C 5/06, A61M 35/00, A45D 34/04, A46B 11/00, B65D 83/00

(54) **APPLIKATIONSVORRICHTUNG**
APPLICATION DEVICE
DISPOSITIF D'APPLICATION

(30) Priorität: 20.08.2007 DE 102007039177; 31.01.2008 DE 202008001427 U
(43) Veröffentlichungstag der Anmeldung: 05.05.2010
(73) Patentinhaber: Sulzer Mixpac AG, 9469 Haag (CH)
(72) Erfinder: SOGARO, Alberto, C., 61476 Kronberg (DE)
(74) Vertreter: advotec.
(86) Internationale Anmeldenummer: PCT/DE2008/001287
(87) Internationale Veröffentlichungsnummer: WO 2009/024117

(56) Entgegenhaltungen:
- EP-A- 1 293 448
- WO-A-01/32242
- DE-A1- 4 016 353
- US-B1- 7 153 053

## Beschreibung

Die Erfindung betrifft eine Applikationsvorrichtung zum Auftragen einer fließfähigen Substanz.

Beispielsweise aus der EP 1 743-700 A1 ist eine Applikationsvorrichtung in Form einer Pipettiervorrichtung bekannt, die aus einer Außenhülse und einer Innenhülse gebildet ist. Die Innenhülse ist topfartig ausgebildet und umfasst einen geschlossenen Boden sowie eine Umfangswand, die mit mehreren Öffnungen versehen ist. Des Weiteren ist die Innenhülse innen an der Umfangswand der Außenhülse gedichtet geführt und zwischen einer Schließstellung und einer Freigabestellung verschiebbar. In der Schließstellung ist in der Innenhülse eine fließfähige Substanz vorgehalten. Beim Verschieben der Innenhülse in die Freigabestellung kann die in der Innenhülse vorgehaltene Substanz durch die Öffnungen an der Umfangswand in die Außenhülse strömen und von dort aus appliziert werden.

Aus der EP 1 293 448 A2 ist eine Applikationsvorrichtung bekannt, die die Merkmale des Oberbegriffs des Anspruchs 1 aufweist.

Der Erfindung liegt die Aufgabe zugrunde, eine Applikationsvorrichtung bereitzustellen, die kompakt ausgebildet ist und bei der eine einfache Aktivierung möglich ist.

Diese Aufgabe ist erfindungsgemäß durch die Applikationsvorrichtung mit den Merkmalen des Schutzanspruchs 1 gelöst.

Gemäß der Erfindung wird mithin eine Applikationsvorrichtung zum Auftragen einer fließfähigen Substanz vorgeschlagen, umfassend eine Außenhülse, die eine äußere Umfangswand und einen äußeren Boden aufweist, eine Innenhülse, die eine innere Umfangswand und einen inneren Boden aufweist und die in der Außenhülse gedichtet geführt ist und zwischen einer Schließstellung und einer Freigabestellung verlagerbar ist, einen Applikator, der zumindest teilweise in die Innenhülse eingreift, wobei der innere Boden der Innenhülse mindestens eine Öffnung aufweist, die in der Schließstellung der Innenhülse verschlossen ist und in der Freigabestellung der Innenhülse zumindest teilweise für die fließfähige Substanz durchlässig ist, wobei ein Innenraum der Innenhülse beim Verfahren der Innenhülse aus der Schließstellung in die Freigabestellung durch Verdrängung mit der in Schließstellung in einem Aufnahmeraum zwischen der Außenhülse und der Innenhülse aufgenommenen, fließfähigen Substanz beaufschlagt wird.

Nach der Erfindung ist an dem Boden der Außen-hülse ein Stift ausgebildet, der bezüglich der Außenhülse axial ausgerichtet ist. Dieser Stift bzw. Dorn dient vorzugsweise dazu, dass er in Schließstellung der Innenhülse die Öffnung im Boden der Innenhülse abdichtet. Der Stift bildet in diesem Fall also die Schließeinrichtung für die Öffnung. Beim Aktivieren der Applikationseinrichtung wird der Dorn durch die Öffnung im Boden der Innenhülse verfahren, so dass die in dem Aufnahmeraum vorgehaltene Substanz in den Innenraum der Innenhülse strömen kann.

Der Kern der Erfindung besteht mithin darin, eine Applikationsvorrichtung bereitzustellen, bei der die Innenhülse als Verdrängungskolben wirkt, so dass bei einem Zusammenschieben von Innenhülse und Außenhülse die in dem Aufnahmeraum vorgehaltene Substanz durch die Öffnung im Boden der Innenhülse in den Innenraum der Innenhülse strömen kann. In der Schließstellung, d. h. im auseinander gezogenen Zustand von Innenhülse und Außenhülse ist die Öffnung im Boden der Innenhülse verschlossen, so dass keine Flüssigkeit aus dem Aufnahmeraum in den Innenraum der Innenhülse strömen kann. Bei der Aktivierung wird die Öffnung freigegeben.

Bei einer bevorzugten Ausführungsform der Vorrichtung nach der Erfindung ist ein Applikator vorgesehen, der zumindest teilweise in die Innenhülse eingreift. Der Applikator wird beim Verfahren der Innenhülse aus der Schließstellung in die Freigabestellung durch Verdrängung mit der in Schließstellung in einem Aufnahmeraum zwischen der Außenhülse und der Innenhülse aufgenommenen, fließfähigen Substanz beaufschlagt.

Um die Öffnung im Boden der Innenhülse wirkungsvoll abdichten und auch freigeben zu können, weist der Stift vorzugsweise an seinem dem Boden der Außenhülse abgewandten Ende einen aufgeweiteten Abschnitt auf, dessen Grundfläche im Wesentlichen mit der Grundfläche der Öffnung korrespondiert. In Schließstellung der Innenhülse dichtet der aufgeweitete Abschnitt die Öffnung ab. Der aufgeweitete Abschnitt bzw. Abschnitt vergrößerten Durchmessers kann natürlich auch ein mittlerer Abschnitt des Stifts sein. In diesem Falle ist die Öffnung auch in Schließstellung vollständig von dem Stift durchgriffen.

Der Stift kann in dem in Schließstellung in Höhe der Öffnung liegenden Bereich, d.h. im aufgeweiteten Abschnitt, eine Dichtlippe oder ein sonstiges Dichtmittel aufweisen. Die Dichtlippe, deren Ausbildung selbst den aufgeweiteten Abschnitt bzw. Bereich vergrößerten Durchmessers des Stifts bewirken kann und die in einer Ringnut des Stifts sitzen kann, kann mit einer Ringnut am Boden der Innenhülse zusammenwirken, die im Bereich des Umfangs der Öffnung der Innenhülse angeordnet ist und in die die Dichtlippe in Schließstellung der Innenhülse eingreift. Alternativ kann auch eine Dichtlippe, die an dem Boden der Innenhülse ausgebildet ist, in Schließstellung an dem aufgeweiteten Abschnitt, d. h. im Bereich vergrößerten Durchmessers des Stifts anliegen. Dort kann dann eine korrespondierende Ringnut ausgebildet sein, in welche die Dichtlippe in Schließstellung einrastet bzw. eingreift.

Bei einer alternativen Ausführungsform ist die Schließeinrichtung von einem als Sollbruchstelle ausgebildeten Bereich des Bodens der Innenhülse gebildet. Die Sollbruchstelle wird bei Verfahren der Innenhülse aus der Schließstellung in die Freigabestellung zur zumindest teilweisen Freigabe der Öffnung von dem Stift durchstoßen. Damit kann die fließfähige Substanz bei Aktivierung der erfindungsgemäß ausgebildeten Vorrichtung aus dem Aufnahmeraum in den Innenraum der Innenhülse strömen.

Vorzugsweise ist die Sollbruchstelle durch eine Materialschwächung oder -verjüngung der Innenhülse in dem entsprechenden Bereich des Bodens der Innenhülse gebildet.

Um die Aktivierung der Applikationsvorrichtung nach der Erfindung zu erleichtern, ist bei einer bevorzugten Ausführungsform die Innenhülse mit einer Greifeinrichtung versehen.

Die Greifeinrichtung ist beispielsweise als Schürze ausgebildet, die die Außenfläche der Umfangswand der Außenhülse zumindest in Freigabestellung zumindest teilweise übergreift. Die Schürze kann an ihrer Außenwand des Weiteren einen bezüglich der Vorrichtung konkav ausgebildeten Bund zum Angriff der Finger eines Benutzers aufweisen. Die Innenhülse kann dann beispielsweise zwischen Zeigefinger und Mittelfinger einer Hand eingeklemmt werden. Durch Druck mit dem Daumen auf den Boden der Außenhülse erfolgt dann eine Aktivierung der Applikationsvorrichtung.

Um in Schließstellung ein ungewolltes Austreten der fließfähigen Substanz aus der Vorrichtung nach der Erfindung zu verhindern, ist es vorteilhaft, wenn zwischen der Innenhülse und der Außenhülse eine Dichteinrichtung angeordnet ist. Beispielsweise umfasst die Dichteinrichtung eine Dichtlippe, die mit mindestens einer Ringnut zusammenwirkt. Beispielsweise ist die Dichtlippe an der Innenwand der Außenhülse und die Ringnut an der Außenwand der Innenhülse angeordnet.

Um einem Benutzer auch einen gefühlten Eindruck der Schließstellung und/oder der Freigabestellung zu vermitteln, kann die Vorrichtung nach der Erfindung eine Rasteinrichtung aufweisen, die die Schließstellung und/oder die Freigabestellung definiert. Diese Rasteinrichtung kann von der oben beschriebenen Dichtlippen/Ringnut-Anordnung gebildet sein.

Die Applikationsvorrichtung nach der Erfindung eignet sich insbesondere zur Anwendung im Bereich der Medizin und/oder der Kosmetik. Beispielsweise kann in dem Aufnahmeraum ein Medikament zur Behandlung eines Hautleidens, beispielsweise eine Flüssigkeit zur Aknebehandlung, vorgehalten werden. Denkbar ist es aber auch, dass die fließfähige Substanz eine im Dentalbereich anwendbare Substanz, beispielsweise Zahnweiß ist.

Der Applikator kann insbesondere als Schwamm, Pinsel oder dergleichen ausgebildet sein. Denkbar ist es aber auch, dass der Applikator als Pipette ausgebildet ist. In diesem Falle kann der Applikator auch einstückig mit der Innenhülse der Applikationsvorrichtung nach der Erfindung gefertigt sein.

Insbesondere bei Ausbildung des Applikators als Schwamm oder als Pinsel ist es vorteilhaft, wenn der Applikator in der Schließstellung der Innenhülse vollständig von der Innenhülse aufgenommen ist und in der Freigabestellung zumindest teilweise über eine Stirnfläche der Innenhülse vorsteht. Durch diese Auslegung kann eine Verschmutzung oder Beschädigung des Applikators in der die Transportstellung darstellenden Schließstellung der Applikationsvorrichtung nach der Erfindung verhindern.

Um den Applikator gegenüber der Innenhülse verschieben und damit beim Aktivieren der Vorrichtung nach der Erfindung aus der Innenhülse schieben zu können, ist es vorteilhaft, wenn der Applikator eine Bodenfläche aufweist, an der der Stift bzw. Dorn angreifen kann. Der Applikator kann aber auch direkt mit dem Stift verbunden sein, welcher an dem äußeren Boden der Außenhülse ausgebildet ist und mit der Öffnung dem inneren Boden der Innenhülse zusammenwirkt.

Um in Schließstellung der Vorrichtung nach der Erfindung ein versehentliches Zusammenschieben von Innenhülse und Außenhülse zu verhindern, kann eine Sicherungseinrichtung vorgesehen sein. Diese ist beispielsweise aus einer Reißlasche gebildet, die in Schließstellung die Innenhülse umgreifend zwischen einem Ringbund der Außenhülse und einem Ringbund oder einer Schürze der Innenhülse angeordnet ist.

Des Weiteren kann an der Innenhülse ein Deckel vorgesehen sein, der in Schließstellung der Vorrichtung nach der Erfindung den in der Innenhülse angeordneten Applikator überdeckt und schützt. Der Deckel ist vorzugsweise einstückig mit der Innenhülse ausgebildet und über ein Filmscharnier oder dergleichen angelenkt. Vorzugsweise ist der Deckel als bistabiles Element ausgebildet, das bis zu einem bestimmten Öffnungswinkel in Schließrichtung und ab dem bestimmten Öffnungswinkel in Öffnungsrichtung vorgespannt ist. Der Deckel schnappt damit bei Erreichen des Öffnungswinkes selbsttätig auf.

Weitere Vorteile und vorteilhafte Ausgestaltungen des Gegenstandes der Erfindung sind der Beschreibung, der Zeichnung und den Schutzansprüchen entnehmbar.

Zwei Ausführungsbeispiele der Applikationsvorrichtung nach der Erfindung sind in der Zeichnung schematisch vereinfacht dargestellt und werden in der nachfolgenden Beschreibung näher erläutert.

Es zeigt:
- Fig. 1: eine perspektivische Ansicht einer Applikationseinrichtung zum Auftragen einer Flüssigkeit;
- Fig. 2: einen Längsschnitt durch die Applikationsvorrichtung nach Fig. 1; und
- Fig. 3: einen Längsschnitt durch eine zweite Ausführungsform der Applikationsvorrichtung.

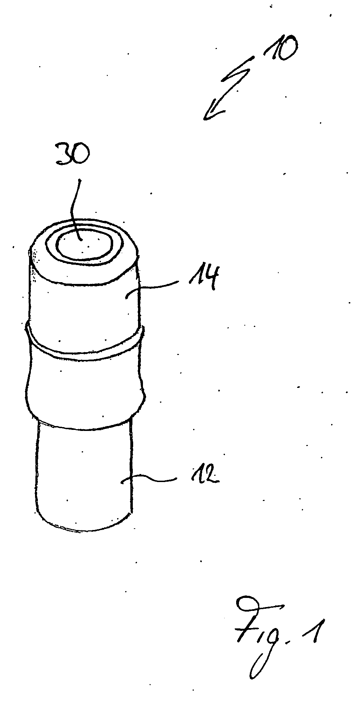

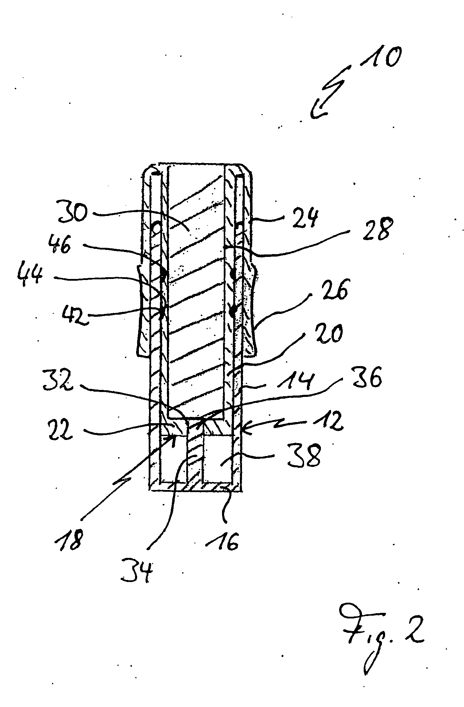

In den Figuren 1 und 2 ist eine Applikationsvorrichtung 10 dargestellt, die stiftartig ausgebildet ist und zum Auftragen einer flüssigen Substanz, wie einem Medikament gegen Akne, auf die Haut eines Patienten dient.

Die Applikationsvorrichtung umfasst eine im Wesentlichen topfartige, aus Kunststoff gefertigte Außenhülse 12, die eine äußere Umfangswand 14 sowie einen äußeren Boden 16 aufweist. An der dem Boden 16 abgewandten Seite ist die zylindrische Außenhülse 12 offen ausgebildet.

Des Weiteren umfasst die Applikationsvorrichtung 10 eine ebenfalls im Wesentlichen topfartig ausgebildete, aus Kunststoff gefertigte Innenhülse 18, die in das offene Ende in der Außenhülse 12 eingesetzt ist und in dieser verschiebbar geführt ist.

Die Innenhülse 18 weist eine innere Umfangwand 20 und einen inneren Boden 22 der Applikationsvorrichtung 10 auf.

An der Innenhülse 18 ist eine Schürze 24 ausgebildet, die an der dem Boden 22 abgewandten Seite an die Innenhülse 18 angeformt ist und die Umfangswand 14 der Außenhülse 12 teilweise übergreift. Die Schürze 24 und die innere Umfangswand 20 der Innenhülse 18 bilden eine ringförmige Aufnahme für einen Endbereich der Umfangswand 14 der Außenhülse 12. An der Schürze 24 ist ein bezüglich der Außenhülse 12 konkav ausgebildeter Ringbund 26 vorgesehen, der als Greifhilfe dient.

Des Weiteren ist ein Innenraum 28 der Innenhülse 18, welcher von der inneren Umfangswand 20 umgeben ist, von einem als Schwamm ausgebildeten Applikator 30 ausgefüllt, mittels dessen die betreffende Flüssigkeit auf die Haut aufgetragen werden kann.

Der innere Boden 22 der Applikationsvorrichtung 10 weist mittig eine kreisförmige Öffnung 32 auf, die mit einem von dem äußeren Boden 16 der Außenhülse 12 vorstehenden Stift 34 zusammenwirkt, und zwar derart, dass ein aufgedickter Endabschnitt 36 des Stifts 34 die Öffnung 32 in einer Schließstellung, welche in Fig. 2 dargestellt ist, dichtet. In einer Freigabestellung, in der die Innenhülse 18 gegenüber der Schließstellung weiter in die Außenhülse 12 verfahren ist, durchgreift der Stift 34 die Öffnung 32 vollständig, so dass zwischen dem Boden 22 und dem Stift 34 eine Ringfläche frei liegt, über welche eine fließfähige Substanz strömen kann.

In der in Fig. 2 dargestellten Schließstellung ist die fließfähige Substanz bzw. das Aknemedikament in einem von dem inneren Boden 22, dem äußeren Boden 16 und der äußeren Umfangswand 12 begrenzten Aufnahmeraum 38 aufgenommen. Beim Verschieben der Innenhülse 18 in die Freigabestellung wirkt die Innenhülse 18 als Verdrängungskolben, der die in dem Aufnahmeraum 38 enthaltene fließfähige Substanz durch die Öffnung 32 in den Schwamm 30 verdrängt, so dass mittels dessen die fließfähige Substanz auf den erkrankten Hautbereich aufgetragen werden kann.

Bei Herstellung der Freigabestellung wird zudem der Endbereich des Schwamms 30 mittels des Stifts 34 über die Stirnseite der Innenhülse 18 hinausgedrückt, so dass eine bequeme Applikation der fließfähigen Substanz möglich ist.

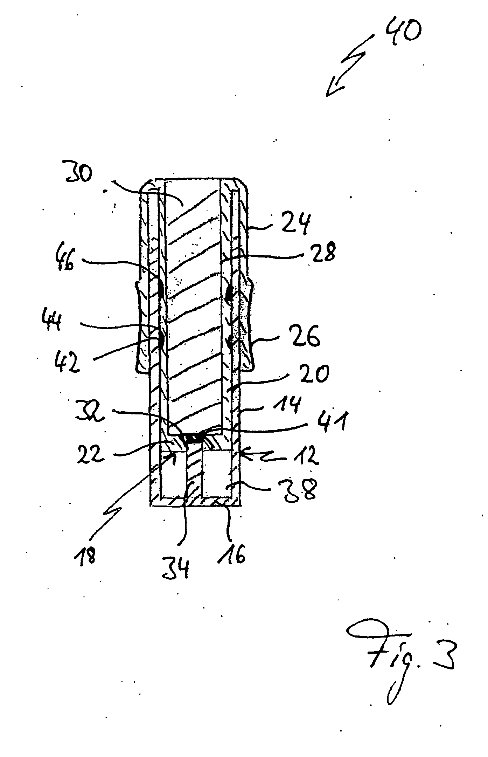

In Fig. 3 ist eine alternative Ausführungsform einer Applikationsvorrichtung zum Auftragen einer fließfähigen Substanz dargestellt. Die Applikationsvorrichtung 40 entspricht im Wesentlichen derjenigen nach den Figuren 1 und 2, unterscheidet sich von dieser aber dadurch, dass nicht der Stift 34 als Schließeinrichtung für die Öffnung 32 dient, sondern die Öffnung 32 mittels eines als Sollbruchstelle ausgebildeten verdünnten Bereichs 41 des inneren Bodens 22 der Innenhülse 18 gebildet ist. Beim Zusammenfahren der Applikationsvorrichtung 40, d. h. beim Herstellen der Freigabestellung, durchstößt der dann stößelartige zylindrisch, ohne Bereich vergrößerten Durchmessers ausgebildete Stift 34 die Sollbruchstelle bzw. den Bereich 41 verringerter Wandstärke, so dass die Öffnung 32 teilweise freigegeben ist und die in dem Aufnahmeraum 38 aufgenommene Flüssigkeit durch Verdrängung in den Schwamm 30 strömen kann.

Die äußere Umfangwand 14 der Außenhülse 12 hat an ihrer Innenseite des Weiteren eine als Ringwulst ausgebildete Dichtlippe 42, die mit zwei Ringnuten 44 und 46 an der Außenseite der Umfangswand 20 der Innenhülse 18 zusammenwirkt. Die Ringnut 44 definiert die Schließstellung der Applikationsvorrichtung 40 und die Ringnut 46 definiert die Freigabestellung der Applikationsvorrichtung 40. Die Ringnuten 44 und 46 haben neben ihrer Dichtfunktion auch eine Rastfunktion für die Dichtlippe 42, damit der Benutzer der Vorrichtung nach der Erfindung ein gefühlten Eindruck beim Erreichen der Freigabestellung vermittelt bekommt.

### Bezugszeichen

- 10: Applikationsvorrichtung
- 12: Außenhülse
- 14: Umfangswand
- 16: Boden
- 18: Innenhülse
- 20: Umfangswand
- 22: Boden
- 24: Schürze
- 26: Ringbund
- 28: Innenraum
- 30: Applikator
- 32: Öffnung
- 34: Stift
- 36: Endabschnitt
- 48: Aufnahmeraum
- 40: Applikationsvorrichtung
- 41: Bereich verringerter Wandstärke
- 42: Dichtlippe
- 44: Ringnut
- 46: Ringnut

## Patentansprüche

1. Applikationsvorrichtung zum Auftragen einer fließfähigen Substanz, umfassend
- eine Außenhülse (12), die eine äußere Umfangswand (14) und einen äußeren Boden (16) aufweist,
- eine Innenhülse (18), die eine innere Umfangswand (20) und einen inneren Boden (22) aufweist und die in der Außenhülse (12) gedichtet geführt ist und zwischen einer Schließstellung und einer Freigabestellung verlagerbar ist,
wobei der innere Boden (22) der Innenhülse (18) mindestens eine Öffnung (32) aufweist, die in der Schließstellung der Innenhülse (18) durch eine Schließeinrichtung (34) verschlossen ist und in der Freigabestellung der Innenhülse (18) zumindest teilweise für die fließfähige Substanz durchlässig ist, wobei ein Innenraum der Innenhülse (18) beim Verfahren der Innenhülse (18) aus der Schließstellung in die Freigabestellung durch Verdrängung mit der in Schließstellung in einem Aufnahmeraum (38) zwischen der Außenhülse (12) und der Innenhülse (18) aufgenommenen, fließfähigen Substanz beaufschlagt wird, **dadurch gekennzeichnet, dass** an dem Boden (16) der Außenhülse (12) ein Stift (34) ausgebildet ist, der bezüglich der Außenhülse (12) axial ausgerichtet ist und in Schließstellung der Innenhülse (18) die Öffnung (32) im Boden (22) der Innenhülse (18) abdichtet.

2. Applikationsvorrichtung nach Anspruch 1, **gekennzeichnet durch** einen Applikator (30), der zumindest teilweise in die Innenhülse (18) eingreift.

3. Applikationsvorrichtung nach Anspruch 1, **dadurch gekennzeichnet, dass** der Stift (34) an seinem dem Boden (16) der Außenhülse (12) abgewandten Ende einen aufgeweiteten Abschnitt (36) aufweist, dessen Grundfläche im Wesentlichen mit der Grundfläche der Öffnung (32) korrespondiert.

4. Applikationsvorrichtung nach Anspruch 1, **dadurch gekennzeichnet, dass** die Schließeinrichtung von einem als Sollbruchstelle ausgebildeten Bereich des Bodens (22) der Innenhülse (18) gebildet ist, wobei die Sollbruchstelle beim Verfahren der Innenhülse (18) aus der Schließstellung in die Freigabestellung zur zumindest teilweisen Freigabe der Öffnung (32) von dem Stift (34) durchstoßen wird.

5. Applikationsvorrichtung nach Anspruch 4, **dadurch gekennzeichnet, dass** die Sollbruchstelle durch eine Materialschwächung gebildet ist.

6. Applikationsvorrichtung nach einem der Ansprüche 1 bis 5, **dadurch gekennzeichnet, dass** die Innenhülse (18) mit einer Greifeinrichtung (24) versehen ist.

7. Applikationsvorrichtung nach Anspruch 6, **dadurch gekennzeichnet, dass** die Greifeinrichtung (24) als Schürze ausgebildet ist, die die Außenfläche der Umfangswand (14) der Außenhülse (12) zumindest in Freigabestellung zumindest teilweise übergreift.

8. Applikationsvorrichtung nach einem der Ansprüche 1 bis 7, **dadurch gekennzeichnet, dass** zwischen der Innenhülse (18) und der Außenhülse (12) eine Dichteinrichtung (42, 44, 46) angeordnet ist.

9. Applikationsvorrichtung nach Anspruch 8, **dadurch gekennzeichnet, dass** die Dichteinrichtung eine Dichtlippe (42) umfasst, die mit mindestens einer Ringnut (44, 46) zusammenwirkt.

10. Applikationsvorrichtung nach einem der Ansprüche 1 bis 9, **gekennzeichnet durch** eine Rasteinrichtung, die die Schließstellung und/oder die Freigabestellung definiert.

11. Applikationsvorrichtung nach einem der Ansprüche 1 bis 10, **dadurch gekennzeichnet, dass** der Applikator (30) einen Schwamm umfasst.

12. Applikationsvorrichtung nach einem der Ansprüche 1 bis 11, **dadurch gekennzeichnet, dass** der Applikator einen Pinsel umfasst.

13. Applikationsvorrichtung nach einem der Ansprüche 1 bis 12, **dadurch gekennzeichnet, dass** der Applikator pipettenartig ausgebildet ist.

14. Applikationsvorrichtung nach einem der Ansprüche 1 bis 13, **dadurch gekennzeichnet, dass** der Applikator (30) in der Schließstellung vollständig von der Innenhülse (18) aufgenommen ist und in der Freigabestellung zumindest teilweise über eine Stirnfläche der Innenhülse (18) vorsteht.

15. Applikationsvorrichtung nach Anspruch 1, **dadurch gekennzeichnet, dass** der Applikator (30) mit dem Stift (34) verbunden ist, der am äußeren Boden (16) der Außenhülse (12) ausgebildet ist.

16. Applikationseinrichtung nach einem der Ansprüche 1 bis 15, **dadurch gekennzeichnet, dass** in Schließstellung zwischen der Innenhülse (18) und der Außenhülse (12) eine vorzugsweise als Reißlasche ausgebildete Sicherungseinrichtung angeordnet ist.

17. Applikationseinrichtung nach einem der Ansprüche 1 bis 16, **gekennzeichnet durch** einen Deckel für den Applikator, der vorzugsweise an der Innenhülse über ein Filmscharnier angelenkt ist.

## Claims

1. Application device to apply a substance capable of flowing, comprising
- an outer sleeve (12) comprising an outer circumferential wall (14) and an outer bottom (16),
- an inner sleeve (18) comprising an inner circumferential wall (20) and an inner bottom (22) and which is lead and sealed in the outer sleeve (12) and movable between a closed position and a release position,
whereby the inner bottom (22) of the inner sleeve (18) has at least one opening (32),which, with the inner sleeve (18) in closed position, is locked by a locking device (34) and, with the inner sleeve (18) in release position, is at least partially permeable for the substance capable of flowing, whereby an internal space of the inner sleeve (18), when moving the inner sleeve (18) from the closed position into the release position, is loaded by displacement with the substance capable to flow which, in closed position, is accepted in a storage space (38) between the outer sleeve (12) and the inner sleeve (18), **characterized in that** there is a locking device (34) at the bottom (16) of the outer sleeve (12) that, referring to the outer sleeve (12), is axially positioned and, with the inner sleeve (18) in closed position, seals the opening (32) in the bottom (22) of the inner sleeve (18).

2. Application device according to claim 1, **characterized by** an applicator (30) which, at least partially, engages with the inner sleeve (18).

3. Application device according to claim 1, **characterized in that** the pin (34) has, at its end not facing the bottom (16) of the outer sleeve (12), a widened section (36) whose surface area basically corresponds with the surface area of the opening (32).

4. Application device according to claim 1, **characterized in that** the locking device is formed by a section of the bottom (22) of the inner sleeve (18) in the form of a predetermined breaking point, whereby the predetermined breaking point is pierced by the pin (34) when moving the inner sleeve (18) from the closed position into the release position to at least partially release the opening (32).

5. Application device according to claim 4, **characterized in that** the predetermined breaking point is formed by material attenuation.

6. Application device according to one of the claims 1 to 5, **characterized in that** the inner sleeve (18) is provided with a gripper (24).

7. Application device according to claim 6, **characterized in that** the gripper (24) is formed as an apron at least partially overlapping the outer surface of the circumferential wall (14) of the outer sleeve (12) at least when in release position.

8. Application device according to one of the claims 1 to 7, **characterized in that** there is a seal (42, 44, 46) between the inner sleeve (18) and the outer sleeve (12).

9. Application device according to claim 8, **characterized in that** the seal is provided with a sealing lip (42) interacting with at least one annular groove (44, 46).

10. Application device according to one of the claims 1 to 9, **characterized by** a snap device which defines the closed position and/or the release position.

11. Application device according to one of the claims 1 to 10, **characterized in that** the applicator (30) comprises a sponge.

12. Application device according to one of the claims 1 to 11, **characterized in that** the applicator comprises a brush.

13. Application device according to one of the claims 1 to 12, **characterized in that** the applicator is formed like a pipette.

14. Application device according to one of the claims 1 to 13, **characterized in that** the applicator (30) is, if in closed position, completely hold by the inner sleeve (18) and, if in release position, at least partially protrudes an end face of the inner sleeve (18).

15. Application device according to claim 1, **characterized in that** the applicator (30) is connected with the pin (34) which is formed at the outer bottom (16) of the outer sleeve (12).

16. Application device according to one of the claims 1 to 15, **characterized in that**, if in closed position, there is a securing device between the inner sleeve (18) and the outer sleeve (12) preferably formed as a pull strap.

17. Application device according to one of the claims 1 to 16, **characterized by** a cap for the applicator, which is preferably pivotable connected to the inner sleeve by means of a film hinge.

## Revendications

1. Dispositif d'application pour l'application d'une substance liquide, comprenant
- un mandrin externe (12) qui présente une paroi périphérique (14) et un fond externe (16),
- un mandrin interne (18) qui présente une paroi périphérique interne (20) et un fond interne (22) et qui est guidé de manière comprimée dans le mandrin externe (12) et qui peut être déplacé entre une position de fermeture et une position de déblocage,
sachant que le fond interne (22) du mandrin interne (18) présente au moins une ouverture qui est verrouillée dans la position fermée du mandrin interne (18) par un dispositif de fermeture et est perméable dans la position de déblocage du mandrin interne (18) au moins partiellement pour la substance liquide, sachant qu'un espace intérieur du mandrin interne (18) est alimenté lors du processus du mandrin interne (18) de la position de fermeture dans la position de déblocage par déplacement, avec la substance liquide recueillie dans la position de fermeture dans un espace de réception (28) entre le mandrin externe (12) et le mandrin interne (18), **caractérisé en ce que** sur le fond (16) du mandrin externe (12) une tige (34) est formée qui est ajustée de manière axiale par rapport au mandrin externe (12) et bouche l'ouverture (32) dans le fond (22) du mandrin interne (18) dans la position de fermeture du mandrin interne (18).

2. Dispositif d'application selon revendication 1, **caractérisé par** un applicateur (30) qui s'emboîte au moins partiellement dans le mandrin interne (18).

3. Dispositif d'application selon revendication 1, **caractérisé en ce que** la tige (34) présente sur son extrémité tournée vers le fond (16) du mandrin externe (12) une section évasée (36) dont la surface correspond essentiellement à la surface de l'ouverture (32).

4. Dispositif d'application selon revendication 1, **caractérisé en ce que** le dispositif de fermeture est formé par une zone du fond (22) du mandrin interne (18) conçue en tant que point de rupture théorique, sachant que le point de rupture théorique est traversé par la tige (34) lors du processus du mandrin interne (18) de la position fermée à la position de déblocage en vue du déblocage au moins partiel de l'ouverture (32).

5. Dispositif d'application selon revendication 4, **caractérisé en ce que** le point de rupture théorique est formé par un affaiblissement de matière.

6. Dispositif d'application selon l'une des revendications 1 à 5, **caractérisée en ce que** le mandrin interne (18) est pourvu d'un dispositif de préhension (24).

7. Dispositif d'application selon revendication 6, **caractérisé en ce que** le dispositif de préhension (24) est formé en tant que jupe, qui recouvre au moins en partie la surface externe de la paroi périphérique (14) du mandrin externe (12) au moins en position de déblocage.

8. Dispositif d'application selon l'une des revendications 1 à 7, **caractérisé en ce qu'**entre le mandrin interne (18) et le mandrin externe (12) un dispositif d'étanchéité (42, 44, 46) est placé.

9. Dispositif d'application selon revendication 8, **caractérisé en ce que** le dispositif d'étanchéité comprend une lèvre d'étanchéité (42) qui agit en interaction avec au moins une rainure annulaire (44, 46).

10. Dispositif d'application selon l'une des revendications 1 à 9, **caractérisé par** un dispositif d'encliquetage qui définit la position de fermeture et / ou la position de déblocage.

11. Dispositif d'application selon l'une des revendications 1 à 10, **caractérisé en ce que** l'applicateur (30) comprend une éponge.

12. Dispositif d'application selon l'une des revendications 1 à 11, **caractérisé en ce que** l'applicateur comprend un pinceau.

13. Dispositif d'application selon l'une des revendications 1 à 12, **caractérisé en ce que** l'applicateur a une formation type pipette.

14. Dispositif d'application selon l'une des revendications 1 à 13, **caractérisé en ce que** l'applicateur (30) est réceptionné dans la position de fermeture entièrement par le mandrin interne (18) et dans la position de déblocage dépasse au moins en partie au-dessus d'une surface frontale du mandrin interne (18).

15. Dispositif d'application selon revendication 1, **caractérisé en ce que** l'applicateur (30) est relié à la tige (34), qui est formée sur le fond externe (16) du mandrin externe (12).

16. Dispositif d'application selon l'une des revendications 1 à 15, **caractérisé en ce que** dans la position de fermeture, un dispositif de sécurité formé de préférence en tant que languette de déchirure est placé entre le mandrin interne (18) et le mandrin externe (12).

17. Dispositif d'application selon l'une des revendications 1 à 16, **caractérisé par** un capuchon pour l'applicateur, qui est articulé de préférence sur le mandrin interne par une charnière-film.
